# EUROPÄISCHE PATENTSCHRIFT

(11) **EP 2 536 448 B1**
(45) Veröffentlichungstag und Bekanntmachung des Hinweises auf die Patenterteilung: **08.01.2014**
(21) Anmeldenummer: 10781803.1
(22) Anmeldetag: 11.11.2010
(51) Int. Cl.: A61M 5/165, A61M 1/00

(54) **KOPPLUNGSTEIL EINER DRAINAGESCHLAUCHEINHEIT**
COUPLING PART FOR A DRAINAGE TUBE UNIT
ÉLÉMENT DE COUPLAGE POUR ASSEMBLAGE À TUYAU DE DRAINAGE

(30) Priorität: 16.02.2010 CH 191102010
(43) Veröffentlichungstag der Anmeldung: 26.12.2012
(73) Patentinhaber: Medela Holding AG, 6340 Baar (CH)
(72) Erfinder: WALTI, Martin, CH-8038 Zürich (CH); JODER, Fabian, CH-8005 Zürich (CH)
(74) Vertreter: Clerc, Natalia
(86) Internationale Anmeldenummer: PCT/CH2010/000283
(87) Internationale Veröffentlichungsnummer: WO 2011/100851

(56) Entgegenhaltungen:
- EP-A1- 2 078 536
- WO-A1-2005/061025
- WO-A1-2008/141470
- WO-A2-2005/035033
- WO-A2-2005/110007
- DE-A1-102006 018 989
- US-A- 3 631 654
- US-A- 4 031 891
- US-A- 4 636 313
- US-A- 5 797 907

## Beschreibung

### TECHNISCHES GEBIET

Die vorliegende Erfindung betrifft ein Kopplungsteil einer Drainageschlaucheinheit gemäss Oberbegriff des Patentanspruchs 1. Sie betrifft insbesondere ein patientenseitiges Anschlussteil.

### STAND DER TECHNIK

Zum Absaugen von Körperflüssigkeiten bzw. -fluiden im medizinischen Bereich, beispielsweise nach chirurgischen Eingriffen, aber auch in der Wunddrainage, der Thoraxdrainage oder beim Absaugen von Körperfetten, werden Drainagepumpsysteme eingesetzt. Diese Drainagepumpsysteme weisen üblicherweise eine Saugpumpe, einen oder mehrere Fluidsammelbehälter und eine Drainageschlauchverbindung zwischen Patient und Fluidsammelbehälter auf. Der Fluidsammelbehälter kann am Gehäuse der Drainagepumpe lösbar befestigt sein oder er kann mit der Pumpe über einen Vakuumschlauch verbunden sein.

Indem mittels der Saug- bzw. Vakuumpumpe im Fluidsammelbehälter ein Unterdruck erzeugt wird, wird das Fluid oder Sekret von einer Kavität des Patienten über den Drainageschlauch in den Sammelbehälter gesaugt und dort gesammelt. Am pumpenseitigen Ausgang des Sammelbehälters angeordnete Filter schützen die Saugpumpe vor einer allfälligen Verunreinigung durch das abgesaugte Fluid.

Es ist ferner bekannt, zusätzlich zur Drainageleitung eine Serviceleitung von der Pumpe zum Patienten zu führen, beispielsweise als Messleitung zur Bestimmung von Strömungs- und Druckdifferenzen oder zur Zuführung von Luft oder Gas, um die Kavität zu spülen. Beispiele hierfür sind US 5 738 656 und US 5 134 996, wobei beide doppel- oder mehrlumige Schläuche offenbaren.

Derartige Messungen über Änderungen des Drucks sind wichtige Hilfsmittel für Ärzte und Pflegepersonal. Druckänderungen während des Absaugens geben beispielsweise Hinweise zur Funktionsfähigkeit der Saugpumpe, zur Dichtheit der Schlauchverbindungen und zum Heilungsprozess.

Ferner offenbart WO 2008/141470 eine Drainageschlaucheinheit zum Absaugen von Körperfluiden mittels einer Saugpumpe. Diese Schlaucheinheit umfasst ein pumpenseitiges Anschlussteil, ein patientenseitiges Anschlussteil, einen Drainageschlauch sowie mindestens einen Serviceschlauch. Der Serviceschlauch kann für die oben genannten Aktionen eingesetzt werden. Die Schläuche sind mit einem ersten Ende im patientenseitigen Anschlussteil und mit einem zweiten Ende im pumpenseitigen Anschlussteil gehalten. Dabei sind die Enden der Schläuche im patientenseitigen Anschlussteil getrennt voneinander in den jeweiligen Anschlussteilen gehalten und über einen Verbindungskanal miteinander verbunden. Diese Drainageschlaucheinheit ist kostengünstig herstellbar und einfach und sicher im Gebrauch.

Es ist ferner bekannt, hydrophobe Filter zu verwenden, um eine Verschmutzung der Saugpumpe zu vermeiden. Beispielsweise offenbart US 4 731 260 einen Fluidbehälter mit einem zylinderförmigen Filtereinsatz. US 4 636 313 offenbart ein Kopplungsteil eines Drainageschlauchs mit einem integrierten Bakterienfilter.

Des Weiteren sind Vorrichtungen zum Entfernen von Gasen aus Flüssigkeiten bekannt, welche gasdurchlässige und flüssigkeitsundurchlässige Filter verwenden. Diese Filter werden von der Flüssigkeit durchflossen, saugen die Flüssigkeit bis zu einem gewissen Grad auf und lassen anschliessend die restliche Flüssigkeit nicht mehr passieren. Beispiele hierfür sind WO 2005/035033, US 3 631 654 und US 4 031 891.

US 5 797 907 zeigt einen Elektrokauter mit einer Saugeinheit gemäß dem Oberbegriff des Anspruchs 1. Diese Vorrichtung weist drei Kanäle und ein Entenschnabelventil auf.

EP 2 078 536 offenbart eine T-förmige Schlauchkupplung mit einem Ventil.

DE 20 2006 018 989 zeigt ein Dreiwegerückschlagventil.

WO 2005/110007 beschreibt ein Druckventil, mit einem ersten und einem zweiten Kanal. Der zweite Kanal ist mit einem federbeaufschlagten Ventilkörper verschliessbar.

### DARSTELLUNG DER ERFINDUNG

Es ist eine Aufgabe der Erfindung, eine Drainageschlaucheinheit zu schaffen, bei welcher sichergestellt ist, dass die Serviceleitung nicht durch abgesaugtes Sekret verstopft werden kann.

Diese Aufgabe löst ein Kopplungsteil einer Drainageschlaucheinheit mit den Merkmalen des Anspruchs 1.

Das erfindungsgemässe Kopplungsteil einer Drainageschlaucheinheit zum Absaugen von Körperfluiden bzw. Sekret mittels einer Saugpumpe umfasst:
einen patientenseitigen Drainageanschluss, eine pumpenseitige Drainagemündung zur Verbindung mit einem Drainageschlauch und einen den Drainageanschluss und die Drainagemündung miteinander verbindenden Drainagekanal; mindestens eine pumpenseitige erste Servicemündung zur Verbindung mit einem Serviceschlauch, eine zweite im Innern des Kopplungsteils endende Servicemündung und einen diese erste und zweite Servicemündungen verbindenden Servicekanal, sowie eine Vorrichtung, welche den Servicekanal vom Drainagekanal trennt. Die Vorrichtung ermöglicht eine Fluidzufuhr vom Servicekanal in den Drainagekanal und verhindert eine Partikel- und Flüssigkeitszufuhr vom Drainagekanal in den Servicekanal. Diese Vorrichtung ist an einer Stelle im Kopplungsteil angeordnet, welche ein Durchströmen des Drainagekanals vom Drainageanschluss zur Drainagemündung ohne Durchdringung der Vorrichtung ermöglicht.

Unter "Durchdringung" wird hier verstanden, dass das Fluid nicht durch das trennende Medium bzw. den trennenden Mechanismus der Vorrichtung strömt. Falls die Vorrichtung einen Bereich aufweist, welcher keine trennende Funktion aufweist, kann das Fluid diese Vorrichtung in diesem Bereich durchdringen. Dieser Bereich kann beispielsweise ein durchgehender Hohlraum sein, wie er in den nachfolgend beschriebenen dritten und vierten Ausführungsbeispielen vorhanden ist.

Dank dieser Vorrichtung im Kopplungsteil wird ermöglicht, dass ein Luftaustausch zwischen Servicekanal und Drainagekanal stattfinden kann. Es wird jedoch frühzeitig verhindert, dass Körperfluide und abgesaugte Gewebeteile in die Serviceleitung gelangen können. Druckmessungen und Spülungen mit Luft oder Gas können somit mittels des Servicekanals jederzeit ungehindert durchgeführt werden.

Erfindungsgemäß verlaufen die pumpenseitige Drainagemündung und die pumpenseitige erste Servicemündung parallel zueinander.

Vorzugsweise handelt es sich bei diesem Kopplungsteil um ein patientenseitiges Anschlussteil oder um einen patientenseitigen Endstecker einer Drainageschlaucheinheit.

Die Vorrichtung kann ein Einwegventil, insbesondere ein Rückschlagventil, ein Filter oder eine Kombination von beidem sein. Das Filter ist luftdurchlässig, aber flüssigkeitsundurchlässig ausgebildet. Vorzugsweise ist es nicht nur hydrophob, sondern auch oliophob ausgebildet.

Vorzugsweise, jedoch nicht zwingend, ist diese Vorrichtung als Membran ausgebildet. In einer bevorzugten Ausführungsform ist die Membran aus einem luftdurchlässigen und flüssigkeitsundurchlässigen Material gefertigt. Dadurch ist eine Druckmessung über die Serviceleitung möglich, ohne dass abgesaugte Flüssigkeit oder abgesaugte Partikel aus dem Drainagekanal in den Servicekanal gelangen können. Die Membran kann vollständig geschlossen ausgebildet sein. Sie kann jedoch auch zusätzlich ein Rückschlagventil aufweisen, beispielsweise indem sie als Entenschnabelventil ausgebildet ist, wobei der Entenschnabel in Richtung Drainagekanal ausgerichtet ist. Weist die Membran zusätzlich zum oben genannten Material dieses Ventil auf, so kann über die Serviceleitung auch eine Flüssigkeit in den Drainagekanal geleitet werden, wobei trotzdem verhindert wird, dass Flüssigkeit in umgekehrter Richtung in den Servicekanal gespült wird.

Es ist auch möglich, dass die Vorrichtung aus einem Rückschlagventil besteht, welches aus einem gasundurchlässigen und flüssigkeitsundurchlässigen Material gefertigt ist. Auch hier kann das Rückschlagventil in Form einer Membran, insbesondere in Form des oben beschriebenen tellerförmigen Grundkörpers mit vorzugsweise zentrisch angeordnetem Entenschnabelventil ausgebildet sein.

In bevorzugten Ausführungsformen weist die Membran eine runde, ovale oder elliptische Grundform auf. Vorzugsweise ist sie einstückig ausgebildet. In einer bevorzugten Ausführungsform weist die Membran einen einstückigen tellerförmigen Grundkörper auf. Falls vorhanden, ist das Rückschlagventil vorzugsweise einstückig an diesen Grundkörper angeformt. Vorzugsweise ist das Rückschlagventil dabei im Zentrum der Membran angeordnet.

Vorzugsweise weist die Membran in ihrem peripheren Bereich mindestens eine umlaufende und in sich geschlossene Dichtlippe auf. Diese dichtet gegenüber einem Membransitz und ermöglicht eine Ventilfunktion.

Vorzugsweise bildet die Membran eine Membranfläche, welche annähernd parallel zu einer Längsachse des Drainagekanals verläuft. Vorzugsweise ist die Membran asymmetrisch zu einer Längsmittelachse des Drainagekanals angeordnet. Dies hat den Vorteil, dass die Vorrichtung dadurch weniger Platz beansprucht.

Vorzugsweise weist die Vorrichtung einen Verbindungskanal auf zur Verbindung der zweiten Servicemündung mit dem Drainagekanal, wobei die Vorrichtung im Bereich dieses Verbindungskanals angeordnet ist. Dies ist insbesondere dann vorteilhaft, wenn der Servicekanal und der Drainagekanal annähernd oder genau parallel zueinander verlaufen.

Die Querschnitte des Servicekanals und, falls vorhanden, des Verbindungskanals sind vorzugsweise beide kleiner als derjenige des Drainagekanals.

Die Membran bzw. eine anders ausgebildete Vorrichtung lässt sich einfach montieren, wenn das Kopplungsteil einen Grundkörper mit einem Sitz zur Aufnahme der Membran bzw. der Vorrichtung und einen den Grundkörper im Bereich des Sitzes nach aussen dicht verschliessenden Deckel aufweist. Die Herstellung ist vereinfacht, wenn der Servicekanal im Deckel angeordnet ist.

In einer bevorzugten Ausführungsform ist zusätzlich eine Zuspritz- und/oder Entnahmeöffnung, vorzugsweise ein Kanal, vorhanden, welche eine Aussenseite des Kopplungsteils mit dem Drainagekanal verbindet. Durch diese Öffnung lässt sich ein Fluid einspritzen oder ein Fluid entnehmen.

In einer ersten Ausführungsform weist das Kopplungsteil genau einen Drainagekanal und genau einen Servicekanal auf. In einer anderen Ausführungsform sind zwei Drainagekanäle und ein einziger Servicekanal vorhanden, wobei der Servicekanal mit beiden Drainagekanälen verbunden ist und wobei die Vorrichtung eine einzige Membran aufweist, welche beide Drainagekanäle von der Serviceleitung trennt.

Anstelle der annähernd planen bzw. tellerförmigen Membran kann die Vorrichtung auch rohrförmig ausgebildet sein. Vorzugsweise verläuft sie dann koaxial zum Drainagekanal. Eine derartige rohrförmige Vorrichtung weist in einer bevorzugten Ausführungsform einen Stützkörper und ein eine Filterfunktion ausübendes Filterelement auf wobei das Filterelement auf dem und/oder im Stützkörper angeordnet ist.

Vorzugsweise ist diese rohrförmige Vorrichtung im Drainagekanal angeordnet, so dass es auch den Verbindungskanal schützt. In einer bevorzugten Ausführungsform weist der Verbindungskanal ein erstes Ende auf, welches in den Drainagekanal mündet, wobei das Filter dieses erste Ende verschliesst.

Um eine Druckmessung zu optimieren, ist zwischen einem äusseren Umfang des Filters und einer Innenwandung des Drainagekanals ein freier Raum ausgebildet. Er kann im Querschnitt ringförmig ausgebildet sein. Vorzugsweise ist er jedoch als Kammer ausgebildet.

Vorzugsweise ist das Filter als Einsatzelement ausbildet, wobei es vorzugsweise über ein saugpumpenseitiges Ende des Drainagekanals in seine einsatzbereite Position im Innern des Kopplungsteils eingeschoben werden kann. Vorzugsweise ist es in seiner Lage eingepresst, so dass diese Presspackung zwischen Filter und Innenwand des Kopplungsteils sicherstellt, dass keine Sekretflüssigkeit in den Servicekanal gelangen kann.

Das Filter weist ein die Filterfunktion ausübendes Filtermaterial auf, welches hydrophob und vorzugsweise auch oliophob ausgebildet ist. Das Filter kann gänzlich aus diesem die Filterfunktion ausübenden Filtermaterial gefertigt sein. Vorzugsweise ist es dann einstückig und/oder selbsttragend ausgebildet.

In einer bevorzugten Ausführungsform weist es jedoch einen Stützkörper und ein die Filterfunktion ausübendes Filterelement auf. Das Filterelement ist dabei auf dem und/oder im Stützkörper angeordnet. Es ist vorzugsweise als Membran ausgebildet. Vorzugsweise ist der Stützkörper hohlzylinderförmig ausgebildet und weist an mindestens einem, vorzugsweise an beiden Enden einen radial nach aussen vorstehenden Flansch auf. Vorzugsweise weist er in Längsrichtung verlaufende Stege oder Verstärkungsrippen auf. Dieser Stützkörper lässt sich einfach und massgetreu herstellen, z. B. aus Kunststoff im Spritzgussverfahren. Des Weiteren lässt sich das Filterelement auf einfache Weise auf ihm befestigen. Beispielsweise lässt es sich innerhalb des Stützkörpers anordnen oder der Stützkörper ist von ihm umwickelt. Das Filterelement lässt sich auch auf die Innen- oder Aussenseite des Stützkörpers aufspritzen. Ist das Filterelement auf der Innenseite des Stützkörpers angebracht, so erhöht sich die Steifigkeit dieses Filterelements.

In einer Variante der Erfindung ist der Drainagekanal über seine gesamte Länge geradlinig ausgebildet. Der Drainagekanal weist vorzugsweise eine Stufe auf, welche als Anschlagfläche für das Filter dient. Vorzugsweise ist in diesem Fall nur ein einziger Drainagekanal vorhanden.

In dieser und in anderen Varianten der Erfindung weist das Filter einen Durchgangskanal auf und ist so im Kopplungsteil angeordnet, dass sein Durchgangskanal während des Absaugens vom Körperfluid durchflossen ist.

In anderen Varianten der Erfindung ist das Filter an einer Stelle im Kopplungsteil angeordnet, welche während des Absaugens vom Körperfluid nicht durchflossen ist. Diese Ausführungsform eignet sich insbesondere auch für Kopplungsteile, welche zwei oder mehrere patientenseitige Drainageanschlüsse aufweisen und somit zur Verzweigung der Drainageleitung dienen.

Das Filter muss nicht zwingend im Drainagekanal angeordnet sein, sondern kann an einer anderen geeigneten Stelle sitzen, solange es den Drainagekanal vom Servicekanal trennt, d.h. verhindert, dass vom Drainagekanal herkommendes Fluid in den Servicekanal gelangen kann, ohne das Filter zu passieren.

Vorzugsweise ist das Kopplungsteil durch ein patientenseitiges Anschlussteil gebildet, wie es in WO 2008/141470 offenbart ist, wobei es erfindungsgemäss mit einer oben beschriebenen Vorrichtung versehen ist. Es ist jedoch auch möglich, anders geformte Kopplungsteile erfindungsgemäss mit einem Filter zu versehen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung eines erfindungsgemässen Kopplungsteils mit angeschlossenem Serviceschlauch und Drainageschlauch in einer ersten Ausführungsform, in einer Ansicht von oben;
- Figur 2: das Kopplungsteil gemäss Figur 1, wobei die Unterseite sichtbar dargestellt ist;
- Figur 3: das Kopplungsteil gemäss Figur 1 in einer Ansicht von unten;
- Figur 4: einen Längsschnitt durch das Kopplungsteil entlang A-A gemäss Figur 3;
- Figur 5: einen Längsschnitt durch das Kopplungsteil entlang B-B gemäss Figur 3;
- Figur 6: einen Querschnitt durch das Kopplungsteil entlang C-C gemäss Figur 4;
- Figur 7: eine perspektivische Darstellung eines erfindungsgemässen Kopplungsteils mit angeschlossenem Serviceschlauch und Drainageschlauch in einer zweiten Ausführungsform, in einer Ansicht von oben;
- Figur 8: das Kopplungsteil gemäss Figur 7, wobei die Unterseite sichtbar dargestellt ist;
- Figur 9: das Kopplungsteil gemäss Figur 7 in einer Ansicht von unten;
- Figur 10: einen Längsschnitt durch das Kopplungsteil entlang A-A gemäss Figur 9;
- Figur 11: einen Längsschnitt durch das Kopplungsteil entlang B-B gemäss Figur 10;
- Figur 12: einen Längsschnitt durch ein erfindungsgemässes Kopplungsteil mit darin angeordneten Schläuchen in einer dritten Ausführungsform;
- Figur 13: einen erfindungsgemässen Stützkörper eines Filters in einer perspektivischen Darstellung gemäss der dritten Ausführungsform;
- Figur 14: einen Längsschnitt durch den Stützkörper gemäss Figur 13 in einer perspektivischen Darstellung mit am Stützkörper angeordnetem Filterelement;
- Figur 15: eine Ansicht eines erfindungsgemässen Kopplungsteils gemäss einer vierten Ausführungsform von oben;
- Figur 16: einen Längsschnitt durch das Kopplungsteil gemäss Figur 15 in einer perspektivischen Darstellung und
- Figur 17: einen Längsschnitt durch das Kopplungsteil gemäss Figur 15.

### BESCHREIBUNG BEVORZUGTER AUSFÜHRUNGSFORMEN

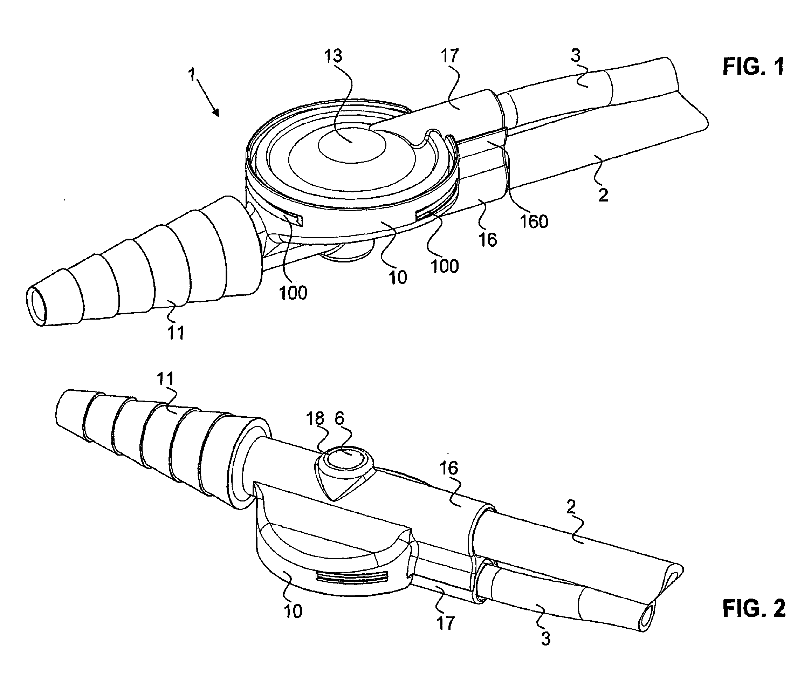

In Figur 1 ist eine erste Ausführungsform des erfindungsgemässen Kopplungsteils 1 mit darin angeordneten Schläuchen 2, 3 dargestellt.

Das Kopplungsteil weist einen Grundkörper 10 mit einem daran angeordneten patientenseitigen Drainageanschluss 11 auf. Vorzugsweise ist das Kopplungsteil 1 aus Kunststoff und im Spritzgussverfahren hergestellt. In dem hier dargestellten bevorzugten Ausführungsbeispiel sind der Grundkörper 10 und der Drainageanschluss 11 gemeinsam einstückig ausgebildet.

Der Grundkörper 10 weist an seinem patientenfernen Ende eine erste Mündung 16 zur Aufnahme des patientenseitigen Endes des Drainageschlauchs 2 auf. Über dieser ersten Mündung 16 ist vorzugsweise eine teilrohrförmige Aufnahme 160 vorhanden, welche nach oben offen ausgebildet ist.

Der Grundkörper 10 ist mit einem Verschlussdeckel 13 verschlossen. Auch dieser ist vorzugsweise aus Kunststoff im Spritzgussverfahren gefertigt. Der Deckel 13 lässt sich vorzugsweise über einen Schnappverschluss mit dem Grundkörper 10 verbinden. Vorzugsweise ist eine einmal erstellte Verbindung nicht zerstörungsfrei lösbar. Der Grundkörper 10 weist hierfür über dem Umfang verteilt angeordnete Fenster 100 auf, in welche radial abstehende Nasen des Deckels 13 eingreifen.

Im Verschlussdeckel 13 ist eine zweite Mündung 17 zur Aufnahme des patientenseitigen Endes des Serviceschlauchs 3 vorhanden. Die zweite Mündung 17 ist rohrförmig ausgebildet und lässt sich formschlüssig in die teilrohrförmige Aufnahme 160 des Grundkörpers 10 einklicken.

Die erste und zweite Mündung 16, 17 verlaufen parallel zueinander, so dass die zwei Schläuche 2, 3 parallel, aber leicht beabstandet zueinander weggeführt sind.

Der Serviceschlauch 3 dient vorzugsweise der Druckmessung im Bereich des Drainageanschlusses 11. Alternativ oder zusätzlich kann der Serviceschlauch 3 auch zur Spülung mit einem gasförmigen oder flüssigen Fluid, insbesondere mit Luft dienen. Sein Durchmesser ist vorzugsweise kleiner als der Durchmesser des Drainageschlauches 2. Beide Schläuche 2, 3 sind vorzugsweise einlumig ausgebildet und sie verlaufen mindestens im Bereich des Kopplungsteils 1 getrennt voneinander. Sie sind vorzugsweise aus Silikon oder PVC gefertigt. Die Schläuche 2, 3 sind in die Mündungen 16, 17 eingesteckt und so darin gehalten. Vorzugsweise sind sie geklebt oder mit dem Kopplungsteil verschweisst. Andere dichtende Fixierungsarten sind ebenfalls möglich. Sie können beispielsweise in einer anderen Ausführungsform auch auf die Mündungen 16, 17 aufgesteckt sein,

Am gegenüberliegenden Ende geht der Grundkörper 10 in den patientenseitigen Drainageanschluss 11 über. Dieser ist vorzugsweise kegelförmig gestuft ausgebildet, wobei er sich zu seinem freien offenen Ende hin verjüngt. Er weist somit im Querschnitt eine Tannenbaumform auf. Der Drainageanschluss 11 verläuft in diesem Beispiel in axialer Flucht zur pumpenseitigen Mündung 16 für den Drainageschlauch 2. Zwischen dieser Mündung 16 und dem patientenseitigen freien Ende des Drainageanschlusses 11 verläuft ein Drainagekanal 12, welcher hier über seine gesamte Länge geradlinig ausgebildet ist.

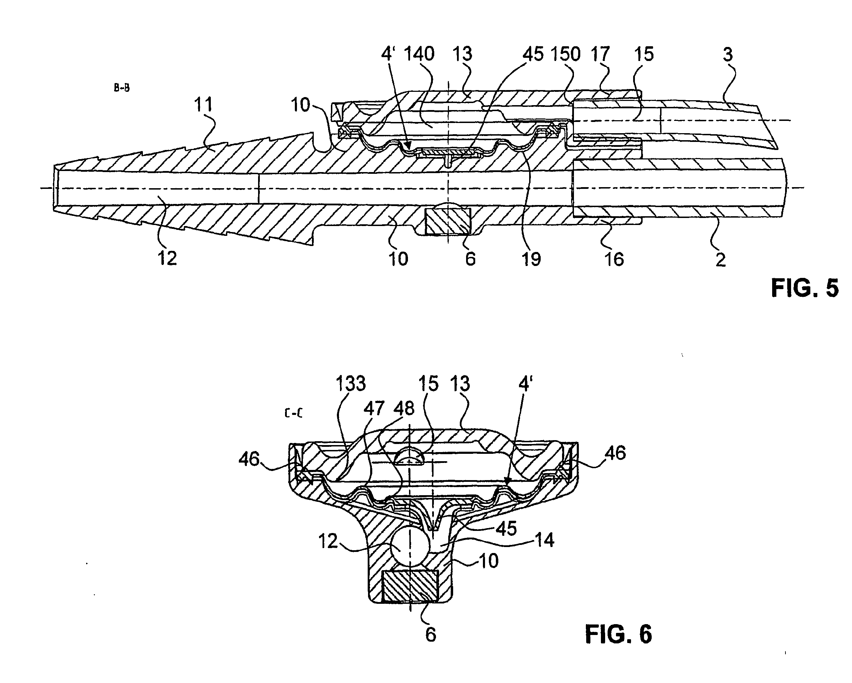

Der Durchmesser der pumpenseitigen Mündung 17 für den Serviceschlauch 3 ist kleiner ausgebildet als derjenige der pumpenseitigen Mündung 16 für den Drainageschlauch 2. Die zweite Mündung 17 ist versetzt zur ersten Mündung 16 angeordnet und sie führt in einen geradlinig und parallel zum Drainagekanal 12 verlaufenden Servicekanal 15. Vorzugsweise verjüngt sich der Servicekanal 15 im Innern des Grundkörpers 10 und bildet so eine Stufe 150. Diese Stufe 150 dient als Anschlag für das patientenseitige Ende des Serviceschlauchs 3. Der Drainagekanal 12 und der Servicekanal 15 sind beispielsweise in Figur 5 erkennbar.

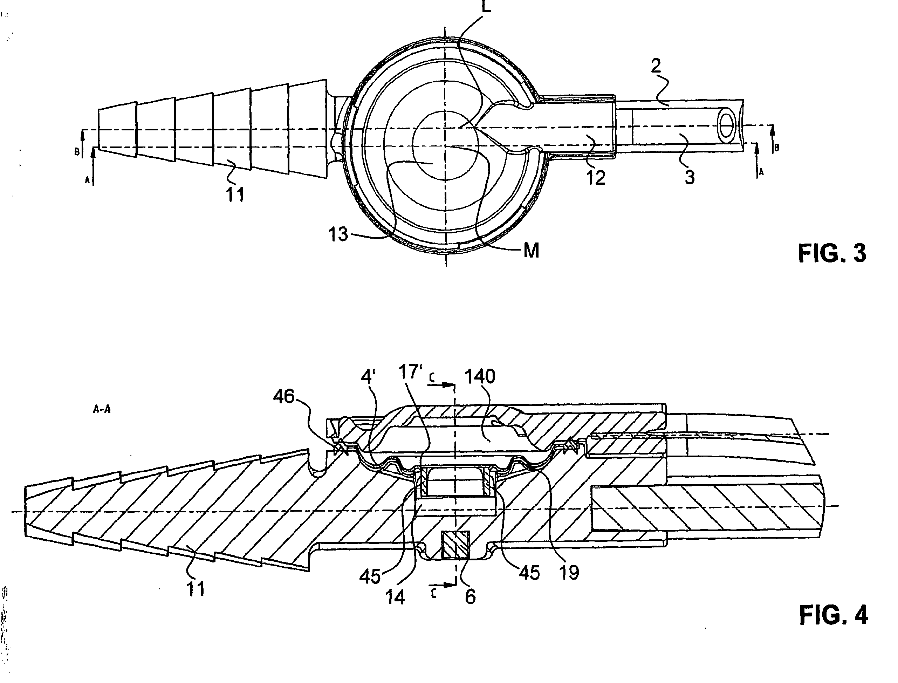

Zwischen dem patientennahen und dem patientenfernen Ende weist der Grundkörper 10 in dieser Ausführungsform einen flachen runden Bereich auf. Der Deckel 13 ist ebenfalls im Wesentlichen flach und rund ausgebildet. Dies ist in Figur 3 gut erkennbar. Dieser runde Bereich und der Deckel 13 bilden eine gemeinsame Kammer 140, welche in den Figuren 4 und 5 gut erkennbar ist. Die Kammer 140 verbindet den Servicekanal 15 mit dem Drainagekanal 12. Sie wird nachfolgend noch im Detail beschrieben.

Wie in den Figuren 4 und 5 erkennbar ist, endet der Servicekanal 15 im Deckel 13 in der Kammer 140, welche in einen Verbindungskanal 14 übergeht. Diese zweite Mündung des Servicekanals 15 ist in den Figuren mit der Bezugsziffer 17' bezeichnet. Der Verbindungskanal 14 verläuft vorzugsweise senkrecht zum Servicekanal 15 und mündet im Drainagekanal 12. Dabei mündet er vorzugsweise in einem rechten Winkel zur Längsachse des Drainagekanals 12. Er ist vorzugsweise geradlinig ausgebildet. Er kann jedoch auch in einem von 90° abweichenden Winkel zum Servicekanal 15 und/oder Drainagekanal 12 stehen und/oder er kann gebogen ausgebildet sein.

Der grundkörperseitige Teil der Kammer 140 ist als Membransitz 19 ausgebildet. Der Membransitz 19 weist einen im peripheren Bereich des Grundkörpers 10 kreisförmigen erhöhten Rand auf. Anschliessend folgt nach innen zum Zentrum hin eine kreisförmige Vertiefung mit einer erneuten erhöhten Rippe. Diese Rippe ist ebenfalls kreisförmig ausgebildet. Im Zentrum M dieser inneren Vertiefung, welches zudem vorzugsweise dem Zentrum M des runden Bereichs des Grundkörpers 10 entspricht, beginnt der Verbindungskanal 14. Er verläuft vorzugsweise senkrecht zum Drainagekanal 12. Sein anderes zweites Ende mündet in den Drainagekanal 12. Sein erstes Ende weist vorzugsweise einen rechteckigen Querschnitt auf.

Die innere Oberfläche des Deckels 13 ist entsprechend ebenfalls gewölbt ausgebildet. Sie weist insbesondere eine nach innen, zum Membransitz hin ragende umlaufende in sich geschlossene Rippe 133 und einen in radialer Richtung zur Rippe 133 äussere plane umlaufende Fläche auf.

In der Kammer 140 ist eine Membran 4' angeordnet. Die Membran 4' ist vorzugsweise aus einem luftdurchlässigen aber flüssigkeitsundurchlässigen Material gefertigt. Sie kann jedoch auch aus einem luft- und flüssigkeitsundurchlässigen Material gefertigt sein. Beispiele für die Materialien sind Elastomere, insbesondere Silikon oder TPE (thermoplastische Elastomere).

Diese Membran 4' weist einen runden Grundriss auf und ist passend zum Membransitz 19 mit umlaufenden, vollständig in sich geschlossenen Ringen 47, 48 versehen. Sie weist in ihrem peripheren Bereich mindestens eine, hier zwei konzentrische Dichtlippen 46 auf. Mindestens eine davon ist relativ scharfkantig ausgebildet. Diese Dichtlippen 46 stützten sich den planen peripheren Bereich des Membransitzes 19 ab und bilden zusammen mit dem radial nach innen nachfolgenden Rippe 133 des Deckels 13 eine dichte Verbindung. Gegen oben, zum Deckel 13 hin, ist in diesem Bereich vorzugsweise ebenfalls eine abstehende, spitze Dichtrippe vorhanden.

Die Membran 4' weist vorzugsweise in ihrem Zentrum ein Schnabelventil 45 (duck valve) auf. Dieses ist vorzugsweise einstückig mit der restlichen Membran 4' gefertigt. Das Schnabelventil 45 ragt in den Verbindungskanal 14 hinein und öffnet somit einen Einlass vom Servicekanal 15 zum Drainagekanal 12 hin, verschliesst jedoch die Gegenrichtung.

Wie in Figur 2 erkennbar ist, ist auf der dem Deckel 13 gegenüberliegenden Seite des Grundkörpers 10 eine Öffnung 18 vorhanden. Diese dient als Zuspritz- und/oder Entnahmeöffnung und ermöglicht während des bestimmungsgemässen Gebrauchs des Kopplungsteils eine Flüssigkeit oder ein Gas in den Drainagekanal 12 einzuspritzen und/oder zu entnehmen. Diese Zuspritz- und/oder Entnahmeöffnung 18 bzw. dieser Kanal ist bei Nichtgebrauch vorzugsweise mit einem Pfropfen 6 dicht verschlossen. Dies ist in den Figuren 4 bis 6 gut erkennbar.

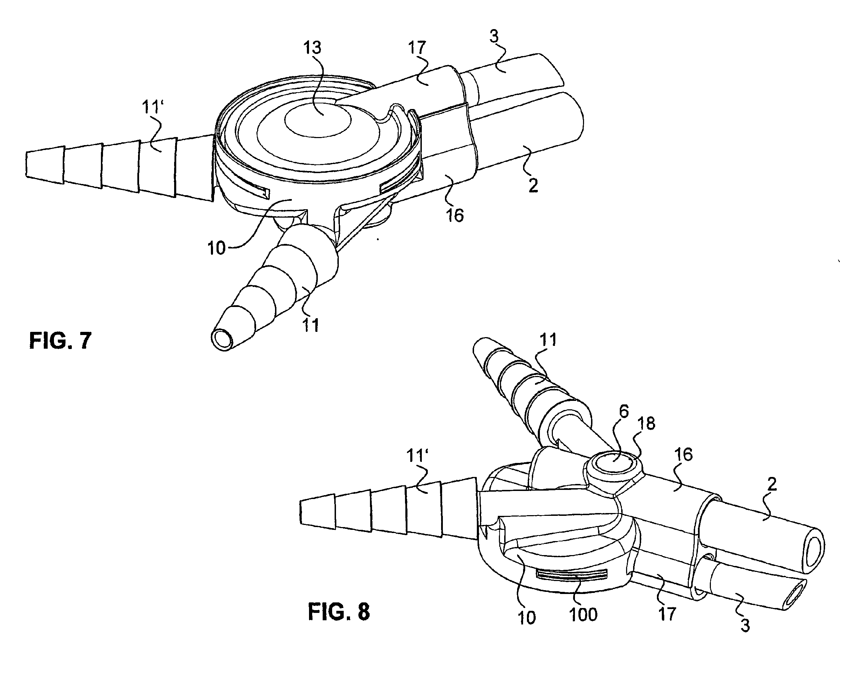

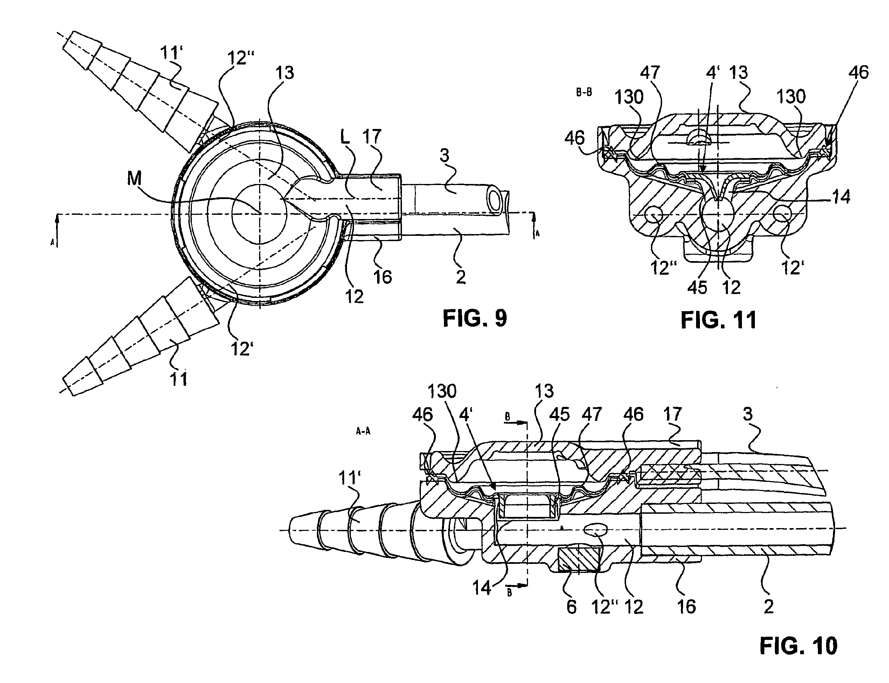

Die Figuren 7 bis 11 zeigen eine zweite Ausführungsform des erfindungsgemässen Kopplungsteils 1. Gleiche Teile sind mit gleichen Bezugszeichen versehen. Auch hier ist der Grundkörper 10 und der den tellerförmigen Bereich des Grundkörpers 10 verschliessenden Deckel 13 vorhanden. Deckel 13 und Grundkörper 10 bilden wiederum die Kammer 140, in welcher die Membran 4' angeordnet ist. Die Kammer 140, insbesondere der Membransitz 19 sowie die Membran 4' selber, sind vorzugsweise gleich ausgebildet wie im oben beschriebenen ersten Ausführungsbeispiel. Des Weiteren ist auch in dieser Ausführungsform vorzugsweise eine mit einem Pfropfen 6 verschliessbare Zuspritz- und/oder Entnahmeöffnung 18 vorhanden.

Der Grundkörper 10 weist in dieser Ausführungsform jedoch drei Enden auf, so dass seine Anschlüsse eine Y-Form bilden. Ein erstes, pumpenseitiges Ende weist wiederum zwei zueinander beabstandet angeordnete Mündungen 16, 17 für den Drainageschlauch 2 und den Serviceschlauch 3 auf. Es sind nun jedoch zwei patientenseitige Enden vorhanden, welche vorzugsweise identisch ausgebildet sind. An jedem Ende befindet sich ein patientenseitiger Drainageanschluss 11, 11'. Beide sind vorzugsweise gleich lang und mit gleichem oder kleinerem Querschnitt wie bzw. als der Drainagekanal 12 ausgebildet. Der pumpenseitige einlumige Drainagekanal 12 verzweigt sich somit innerhalb des Grundkörpers 10 in zwei vorzugsweise identische Drainagekanaläste 12', 12". Die Abzweigung erfolgt vorzugsweise in Absaugrichtung vor der Mündung 17' des Servicekanals 14 in den Drainagekanal 12. D. h. der Drainagekanal 12 im Grundkörper 10 weist einen über die Abzweigung der Drainagekanaläste 12', 12" weitergehenden Ast auf, welcher vorzugsweise die patientenseitige geradlinige Verlängerung des Drainagekanals 12 bildet. Vorzugsweise ist diese Verlängerung lang genug, so dass sich das Rückschlagventil 45 vollständig im Bereich dieser Verlängerung befindet.

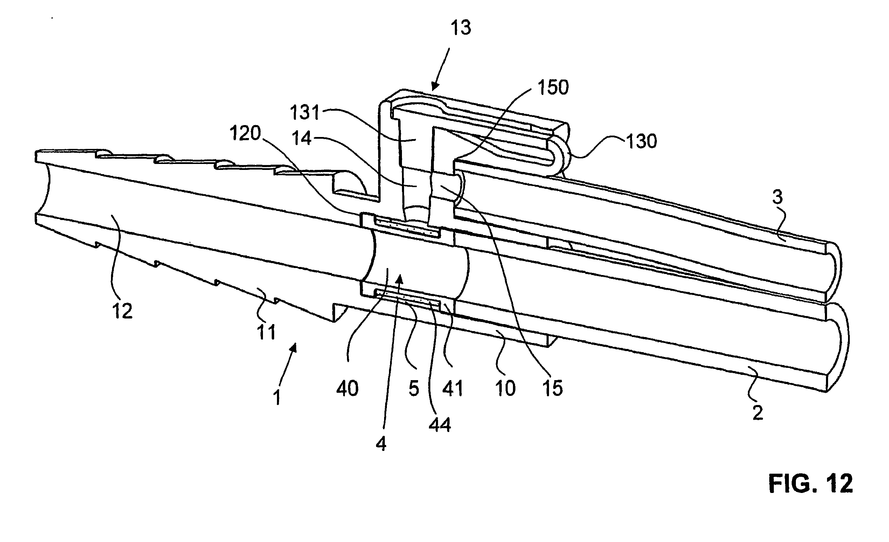

In Figur 12 ist eine dritte Ausführungsform des erfindungsgemässen Kopplungsteils 1 mit darin angeordneten Schläuchen 2 und 3 dargestellt. Auch hier sind gleiche Teile mit gleichen Bezugszeichen versehen und werden nicht mehr im Detail wiederholt. Das Kopplungsteil weist den Grundkörper 10 und den daran angeordneten patientenseitigen Drainageanschluss 11 auf. Grundkörper 10 und Drainageanschluss 11 sind wiederum vorzugsweise gemeinsam einstückig ausgebildet.

In diesem Beispiel sind nun beide Mündungen für patientenseitige Enden des Drainageschlauchs 2 und des Serviceschlauchs 3 im Grundkörper 10 ausgebildet. Der Durchmesser des Serviceschlauchs 3 ist wiederum vorzugsweise kleiner ausgebildet als der Durchmesser des Drainageschlauches 2. Beide Schläuche 2, 3 sind vorzugsweise einlumig ausgebildet und sie verlaufen mindestens im Bereich des Kopplungsteils 1 getrennt voneinander.

Am gegenüberliegenden Ende geht der Grundkörper 10 in den patientenseitigen Drainageanschluss 11 über. Dieser ist wiederum vorzugsweise kegelförmig gestuft ausgebildet. Der Drainageanschluss 11 verläuft in axialer Flucht zur pumpenseitigen Mündung für den Drainageschlauch 2. Zwischen dieser Mündung und dem patientenseitigen freien Ende des Drainageanschlusses 11 verläuft der Drainagekanal 12, welcher vorzugsweise über seine gesamte Länge geradlinig ausgebildet ist.

Der Durchmesser der pumpenseitigen Mündung für den Serviceschlauch 3 ist entsprechend kleiner ausgebildet als derjenige der pumpenseitigen Mündung für den Drainageschlauch 2. Diese zweite Mündung ist axial versetzt zur ersten Mündung im Grundkörper 10 angeordnet und sie führt in den geradlinig und parallel zum Drainagekanal 12 verlaufenden Servicekanal 15. Vorzugsweise verjüngt sich der Servicekanal 15 im Innern des Grundkörpers 10 und bildet so eine Stufe 150. Diese Stufe 150 dient als Anschlag für das patientenseitige Ende des Serviceschlauchs 3.

Der Servicekanal 15 endet im Grundkörper 10 und mündet dort in den Verbindungskanal 14, welcher vorzugsweise senkrecht zum Servicekanal 15 verläuft. Der Verbindungskanal 14 weist denselben oder einen kleineren Durchmesser auf als der Servicekanal 15. Er endet einerseits im Drainagekanal 12, wobei er vorzugsweise senkrecht zur Längsrichtung des Drainagekanals 12 in diesen mündet. Sein anderes Ende bildet eine Öffnung nach aussen, welche vorzugsweise senkrecht zu den pumpenseitigen Mündungen des Drainagekanals 12 und des Servicekanals 15 verläuft. Es ist auch möglich, dass der Verbindungskanal einen Winkel aufweist, dass er gebogen ausgebildet ist und/oder dass er in einem anderen Winkel in den Drainagekanal 12 oder den Servicekanal 15 mündet.

Die nach aussen führende Öffnung des Verbindungskanals 14 ist mit einem Verschlussdeckel 13 verschlossen. In der Figur 12 ist er im geschlossenen Zustand dargestellt. Vorzugsweise ist er einstückig mit dem restlichen Kopplungsteil 1 hergestellt. Er weist ein Verschlussband 130 auf, welches mit einem ersten Ende am Grundkörper 10 angeformt ist. An einem zweiten Ende ist am Verschlussband 130 ein Stopfen 131 angeformt, welches in die nach aussen führende Öffnung des Verbindungskanals 14 einsteckbar und dieser so luft- und flüssigkeitsdicht verschliessbar ist.

Anstelle einer Membran ist nun im Kopplungssteil 1 ein Filter 4 angeordnet, welches luftdurchlässig, aber flüssigkeitsdicht, d.h. für Flüssigkeiten nicht durchlässig, ausgebildet ist. Dieses Filter 4 trennt den Servicekanal 15 vom Drainagekanal 12. Fluid, welches von einer mit dem Drainageschlauch 2 verbundenen Saugpumpe aus einer Kavität des Patienten abgesaugt wird, fliesst ungehindert durch den Drainagekanal 12, ohne das Filter zu durchdringen. Das gleiche Fluid kann jedoch nicht direkt in den Servicekanal 15 gelangen, sondern muss in diesem Fall durch das Filter fliessen. Dadurch kann verhindert werden, dass abgesaugte Flüssigkeit, Gewebepartikel oder andere Festteilchen in den Servicekanal 15 und den Serviceschlauch 3 gelangen und diese verstopfen können.

Vorzugsweise ist das Filter 4 im Drainagekanal 12 angeordnet, wobei es die Mündung des Verbindungskanals 14 in den Drainagekanal 12 verschliesst bzw. in diesem Bereich angeordnet ist. In diesem Beispiel ist ein Abstand zwischen Filter 4 und Mündung vorhanden. Der dadurch entstehende Freiraum 5 ist vorzugsweise ringförmig ausgebildet und erstreckt sich radial um die gesamte Längsmittelachse L des Drainagekanals 12. Dieser Abstand erleichtert die Druckmessung im Drainagekanal 12, wobei der Druck in diesem Bereich gemessen wird.

Vorzugsweise ist das Filter 4 als Einsatzelement ausgebildet, welches sich Grundkörper 10 oder im Drainageanschluss 11 befestigen lässt. Vorzugsweise ist es hohlzylinderförmig bzw. rohrförmig ausgebildet, wobei es im montierten Zustand koaxial zum Drainagekanal 12 verläuft. Das Filter 4 weist ein die Filterfunktion ausübendes Material auf, hier ein Filterelement 44, welches sich entlang des Mantels der Hohlzylinderform erstreckt. Dadurch fliesst aus der Kavität des Patienten abgesaugtes Fluid durch den Drainagekanal 12, ohne das Filterelement 44 zu durchdringen. Der ringförmige Freiraum 5, welcher mit dem Servicekanal 15 verbunden ist, befindet sich jedoch auf der anderen Seite des Filterelements 44.

Im Übergang vom Grundkörper 10 zum patientenseitigen Drainageanschluss 11 weist der Drainagekanal 12 eine Stufe 120 auf. Der Drainagekanal 12 im Drainageanschlussteil 11 weist deshalb einen kleineren Durchmesser auf als der Abschnitt des Drainagekanals 12 im Grundkörper 10. Diese Stufe 120 dient als Anschlag für das Filter 4. Der restliche Abschnitt des Drainagekanals 12, also der pumpenseitige Abschnitt, weist vorzugsweise einen Innendurchmesser auf, welcher mindestens gleich gross ist wie der Aussendurchmesser des Filters 4. Dadurch lässt sich das Filter 4 auf einfache Art und Weise von der pumpenseitigen Mündung her durch den Drainagekanal 12 bis zum Anschlag 120 schieben bzw. hineinpressen. Vorzugsweise dient dann das pumpenseitige Ende des Filters 4 als Anschlag für den Drainageschlauch 2. Vorzugsweise ist eine Presspackung zwischen Filter 4 und Innenwandung des restlichen Kopplungsteils 1 vorhanden, so dass keine Sekretflüssigkeit in den Servicekanal 15 gelangen kann und somit eine Druckmessung in diesem Kanal unbeeinflusst durchgeführt werden kann.

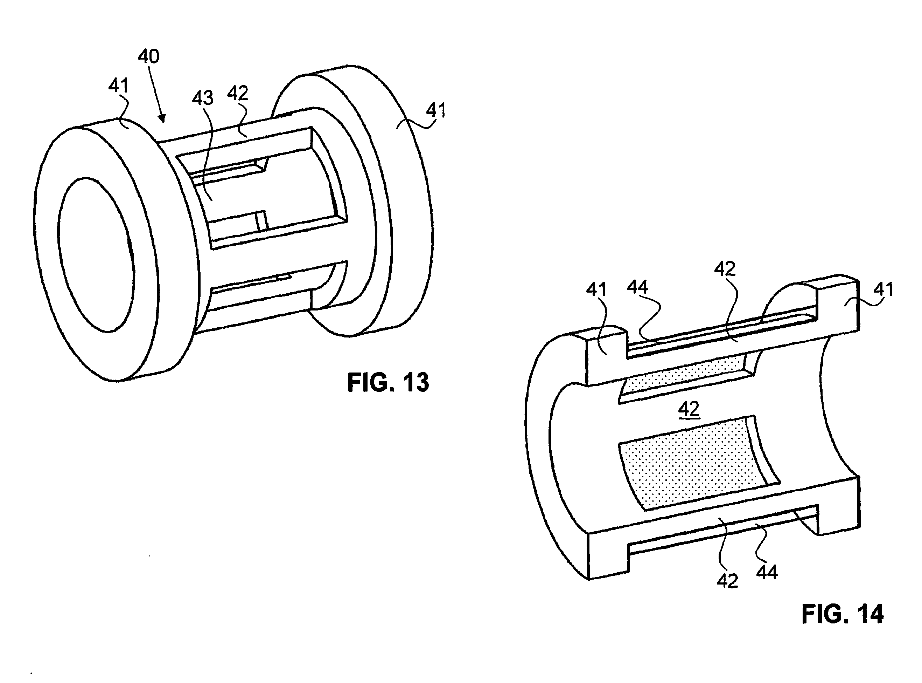

Das Filter 4 kann vollständig aus dem Filterelement 44 bestehen, welches selbsttragend ausgebildet ist. Vorzugsweise weist das Filter 4 jedoch, wie hier dargestellt, einen formstabilen Stützkörper 40 auf, an welchem das Filterelement 44 angeordnet ist. Eine bevorzugte Ausführungsform des Stützkörpers 40 ist in Figur 13 dargestellt. Er ist hantelförmig gestaltet und weist zwei einander gegenüberliegende Endflansche 41 mit erweitertem Durchmesser auf. Diese zwei Endflansche 41 sind über Längsstege 42 miteinander verbunden, wobei zwischen den Längsstegen 42 durchgehende Fenster 43 ausgebildet sind. Durch diese Fenster 43 kann Luft in den Verbindungskanal 14 und zum Servicekanal 15 gelangen. Der Stützkörper 40 ist vorzugsweise aus Kunststoff oder Metall gefertigt. Vorzugsweise ist er einstückig ausgebildet.

In Figur 14 ist eine mögliche Variante zur Anordnung des Filterelements 44, welches die Filterfunktion ausübt, dargestellt. Der Filterelement 44 ist zwischen den zwei Endflanschen 41 um die Längsstege 42 gewickelt, so dass es die Fenster 43 verschliesst. Es bildet somit gemeinsam mit den Endflanschen 41 den äusseren Umfang des Filtereinsatzes 4. Vorzugsweise weist das Filterelement 44 im montieren Zustand einen kleineren äusseren Durchmesser auf als der Stützkörper 40. Es weist im abgewickelten Zustand vorzugsweise eine rechteckige Grundform auf.

Das Filterelement 44 ist vorzugsweise aus einem hydrophoben und insbesondere aus einem zusätzlich oliophoben Material hergestellt. Vorzugsweise ist das Material relativ biegeweich, so dass es auf einfache Art und Weise um den Stützkörper 40 gewickelt werden kann.

Alternativ oder zusätzlich kann das Filterelement 44 auch auf der Innenseite des Stützkörpers 40 angeordnet sein. Dadurch weist es eine erhöhte Stabilität auf.

Das Filterelement 44 kann aufgespritzt, angeklebt, verschweisst oder anderweitig mit dem Stützkörper 40 verbunden sein.

Anstelle von Stegen und Fenstern können auch eine Vielzahl von Schlitzen oder anders geformten Öffnungen in einem an sich geschlossenen Grundkörper des Stützkörpers vorgesehen sein. Diese Schlitze oder Öffnungen sind dann vom Filterelement überdeckt.

Das Filter, d.h. der Stützkörper 40 bzw. das selbsttragende Filterelement 44, kann anstelle eines runden Querschnitts auch eine andere Querschnittsform aufweisen. Beispielsweise kann es oval oder polygonförmig sein, insbesondere vier- oder dreieckig.

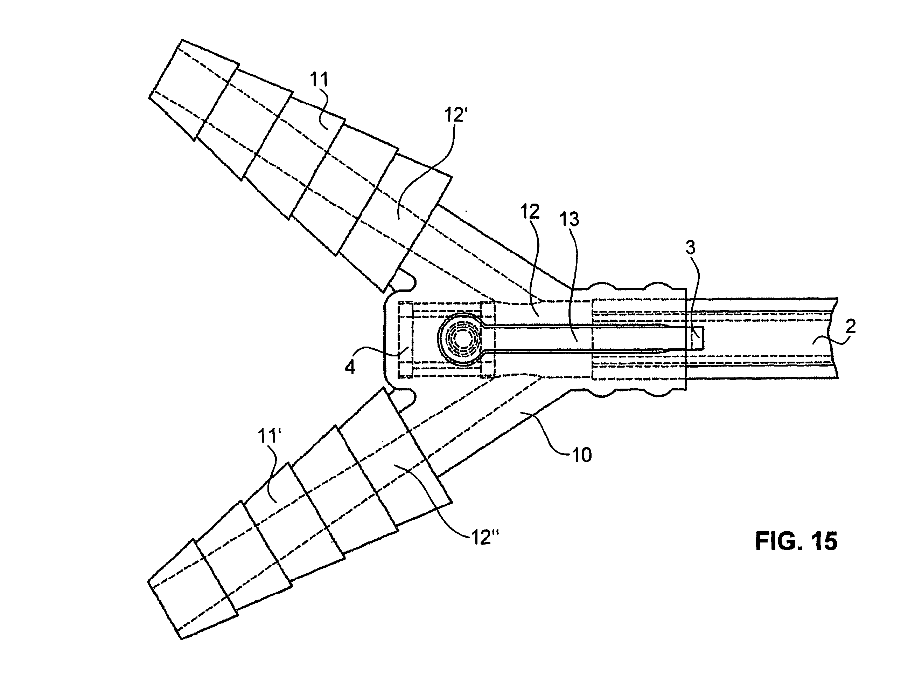

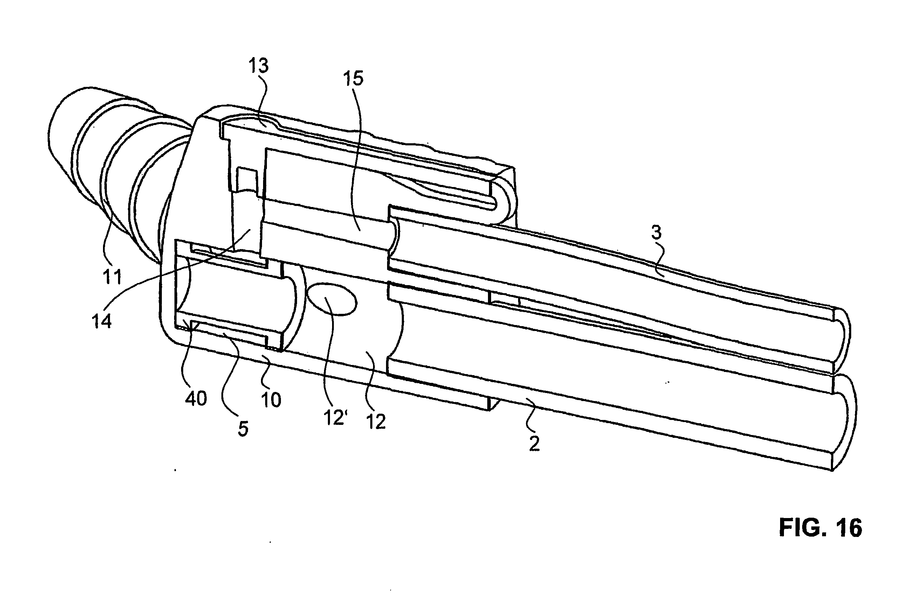

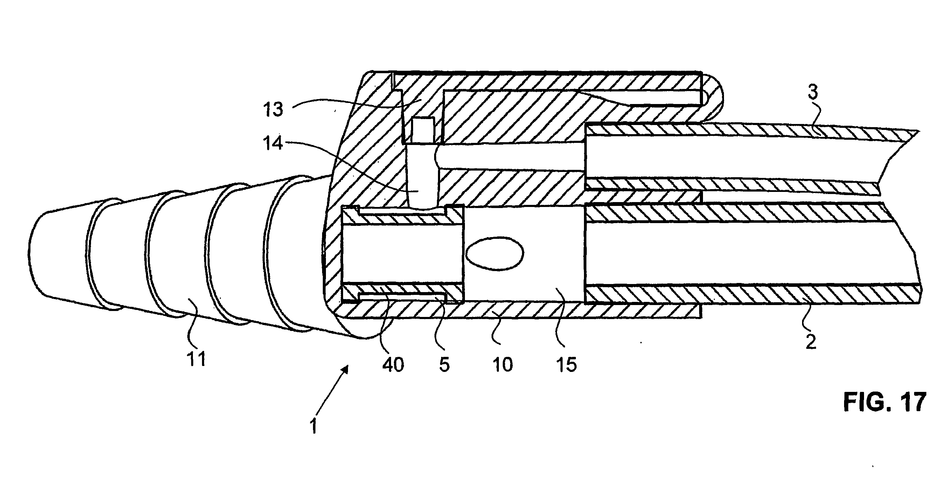

Derselbe Filtereinsatz 4 lässt sich auch in einer anderen Ausführungsform des erfindungsgemässen Kopplungsteils 1 verwenden. Dies ist in den Figuren 15 bis 17 dargestellt. Der grundsätzliche Aufbau entspricht dem bereits beschriebenen dritten Ausführungsbeispiel, so dass gleiche Teile mit gleichen Bezugszeichen versehen sind. Auch hier ist ein Grundkörper 10 vorhanden. Dieser weist jedoch drei Enden auf und ist somit y-förmig ausgebildet. Ein erstes, pumpenseitiges Ende weist wiederum zwei zueinander beabstandet angeordnete Mündungen für einen Drainageschlauch 2 und einen Serviceschlauch 3 auf (siehe Figuren 16 und 17). Es sind nun jedoch zwei patientenseitige Enden vorhanden, welche vorzugsweise identisch ausgebildet sind. An jedem Ende befindet sich ein patientenseitiger Drainageanschluss 11, 11'. Beide sind vorzugsweise gleich lang und mit gleichem Querschnitt des Drainagekanals 12', 12" ausgebildet. Der pumpenseitige einlumige Drainagekanal 12 verzweigt sich somit innerhalb des Grundkörpers 10 in zwei vorzugsweise identische Drainagekanaläste 12', 12". Die Abzweigung erfolgt vorzugsweise in Absaugrichtung vor der Mündung des Servicekanals 14 in den Drainagekanal 12. D. h. der Drainagekanal 12 im Grundkörper 10 weist einen über die Abzweigung der Drainagekanaläste 12', 12" weitergehenden Ast auf, welcher vorzugsweise die patientenseitige geradlinige Verlängerung des Drainagekanals 12 bildet. Vorzugsweise ist diese Verlängerung lang genug, um das Filter 4 aufzunehmen, ohne dass dieses Filter 4 in den Bereich der Abzweigungen zu den Drainagekanalälsten 12', 12" hinein ragt. Dadurch fliesst abgesaugtes Fluid nicht durch das Filter 4. Durch diese Anordnung wird eine frühzeitige Sättigung des Filtermaterials verhindert.

Auch hier lässt sich das Filter 4 wieder über die pumpenseitige Mündung des Drainagekanals 12 in seine Gebrauchslage einschieben bzw. einpressen. Es lässt sich jedoch auch von einem patientenseitigen, zwischen den zwei Drainageanschlüssen 11, 11' liegenden patientenseitigen Ende des Gründkörpers 10 in diesen einschieben (diese Variante ist hier nicht dargestellt).

Anstelle des oben genannten Filtereinsatzes lassen sich in beiden anhand der Figuren 12 bis 17 beschriebenen Ausführungsformen auch andere Filter verwenden. Des Weiteren lassen sich auch andere Formen von anhand der obigen Figuren 1 bis 11 beschriebenen Membranen bzw. andere Arten von Rückschlagventilen einsetzen. Des Weiteren lassen sich das beschriebene Filter und die beschriebene Membran auch in weiteren Ausführungsformen verwenden.

Das erfindungsgemässe Kopplungsteil mit seiner Vorrichtung zur Trennung des Servicekanals vom Drainagekanal ermöglicht eine zuverlässige Druckmessung.

### BEZUGSZEICHENLISTE

| | | | |
|---|---|---|---|
| 1 | Kopplungsteil | | |
| 10 | Grundkörper | 2 | Drainageschlauch |
| 100 | Fenster | | |
| 11, 11' | patientenseitiger Drainageanschluss | 3 | Serviceschlauch |
| 12 | Drainagekanal | 4 | Filter |
| 12', 12" | Drainagekanalast | 4' | Membran |
| 120 | Stufe | 40 | Stützkörper |
| 13 | Verschlussdeckel | 41 | Endflansch |
| 130 | Verschlussband | 42 | Längssteg |
| 131 | Stopfen | 43 | Fenster |
| 133 | Rippe | 44 | Filterelement |
| 14 | Verbindungskanal | 45 | Schnabelventil |
| 140 | Kammer | 46 | Dichtlippe |
| 15 | Servicekanal | 47 | Ring |
| 150 | Stufe | 48 | Ring |
| 16 | Mündung des Drainagekanals | | |
| 160 | teilrohrförmige Aufnahme | 5 | Freiraum |
| 17 | erste Mündung des Servicekanals | | |
| | | 6 | Pfropfen |
| 17' | zweite Mündung des Servicekanals | | |
| | | L | Längsmittelachse |
| 18 | Zuspritz- und/oder Entnahmeöffnung | M | Mittelpunkt |
| 19 | Membransitz | | |

## Patentansprüche

1. Kopplungsteil (1) einer Drainageschlaucheinheit zum Absaugen von Körperfluiden mittels einer Saugpumpe, umfassend:
einen patientenseitigen Drainageanschluss (11), eine pumpenseitige Drainagemündung (16) zur Verbindung mit einem Drainageschlauch (2) und einen den Drainageanschluss (11) und die Drainagemündung (16) miteinander verbindenden Drainagekanal (12);
eine pumpenseitige erste Servicemündung (17) zur Verbindung mit einem Serviceschlauch (3), eine zweite im Innern des Kopplungsteils (1) endende Servicemündung (17') und einen diese erste und zweite Servicemündungen (17, 17') verbindenden Servicekanal (15), sowie
eine Vorrichtung (4, 4'), welche den Servicekanal (15) vom Drainagekanal (12) trennt, wobei die Vorrichtung eine Fluidzufuhr vom Servicekanal (15) in den Drainagekanal (12) ermöglicht und eine Partikel- und Flüssigkeitszufuhr vom Drainagekanal (12) in den Servicekanal (15) verhindert und wobei diese Vorrichtung (4, 4') an einer Stelle im Kopplungsteil (1) angeordnet ist, welche ein Durchströmen des Drainagekanals (12) vom Drainageanschluss (11) zur Drainagemündung (16) ohne Durchdringung der Vorrichtung (4, 4') ermöglicht,
**dadurch gekennzeichnet, dass** die pumpenseitige Drainagemündung (16) und die pumpenseitige erste Servicemündung (17) parallel zueinander verlaufen.

2. Kopplungsteil nach Anspruch 1, wobei ein Verbindungskanal (14) vorhanden ist zur Verbindung der zweiten Servicemündung (17') mit dem Drainagekanal (12) und wobei die Vorrichtung (4, 4') im Bereich dieses Verbindungskanals (14) angeordnet ist.

3. Kopplungsteil nach einem der Ansprüche 1 oder 2, wobei der Servicekanal (15) und der Drainagekanal (12) annähernd oder genau parallel zueinander verlaufen.

4. Kopplungsteil nach einem der Ansprüche 1 bis 3, wobei die Vorrichtung (4, 4') ein Filter aufweist, welches luftdurchlässig und flüssigkeitsundurchlässig ausgebildet ist.

5. Kopplungsteil nach einem der Ansprüche 1 bis 4, wobei die Vorrichtung (4') ein Einwegventil (45) aufweist, insbesondere ein Entenschnabelventil.

6. Kopplungsteil nach einem der Ansprüche 1 bis 5, wobei die Vorrichtung (4') eine Membran ist, wobei die Membran als Filter und/oder als Rückschlagventil wirkt.

7. Kopplungsteil nach Anspruch 6, wobei die Membran (4') rund ausgebildet ist und in ihrem peripheren Bereich mindestens eine umlaufende in sich geschlossene Dichtlippe (46) aufweist.

8. Kopplungsteil nach einem der Ansprüche 6 oder 7, wobei es einen Grundkörper (10) mit einem Membransitz (19) zur Aufnahme der Membran (4') und einen den Grundkörper (10) im Bereich des Membransitzes (19) nach aussen dicht verschliessenden Deckel (13) aufweist.

9. Kopplungsteil nach Anspruch 8, wobei der Servicekanal (15) im Deckel (13) angeordnet ist.

10. Kopplungsteil nach einem der Ansprüche 6 bis 9, wobei die Membran (4') eine Membranfläche bildet, welche annähernd parallel zu einer Längsachse (L) des Drainagekanals (12) verläuft.

11. Kopplungsteil nach einem der Ansprüche 6 bis 10, wobei die Membran (4') asymmetrisch zu einer Längsmittelachse (L) des Drainagekanals (12) angeordnet ist.

12. Kopplungsteil nach einem der Ansprüche 1 bis 11, wobei ferner eine Zuspritz- und/oder Entnahmeöffnung (18) vorhanden ist, welche eine Aussenseite des Kopplungsteils (1) mit dem Drainagekanal (12) verbindet.

13. Kopplungsteil nach einem der Ansprüche 1 bis 12, wobei zwei Drainagekanäle (12, 12') und ein einziger Servicekanal (15) vorhanden sind, wobei der Servicekanal (15) mit beiden Drainagekanälen (12, 12') verbunden ist und wobei die Vorrichtung (4, 4') eine einzige Membran bzw. ein einziges Filter aufweist, welche beide Drainagekanäle (12, 12') von der Serviceleitung (15) trennt.

14. Kopplungsteil nach einem der Ansprüche 1 bis 4, wobei die Vorrichtung (4) rohrförmig ausgebildet ist und koaxial zum Drainagekanal (12) verläuft.

15. Kopplungsteil nach Anspruch 14, wobei die Vorrichtung (4) einen Stützkörper (40) und ein eine Filterfunktion ausübendes Filterelement (44) aufweist, wobei das Filterelement (44) auf dem und/oder im Stützkörper (40) angeordnet ist.

## Claims

1. A coupling part (1) of a drainage tube unit for aspirating body fluids by means of a suction pump, comprising:
a patient-side drainage connection (11), a pump-side drainage orifice (16) for connection to a drainage tube (2), and a drainage channel (12) connecting the drainage connection (11) and the drainage orifice (16) to each other;
a pump-side first service orifice (17) for connection to a service tube (3), a second service orifice (17') ending in the interior of the coupling part, and a service channel (15) connecting these first and second service orifices (17, 17'), and
a device (4, 4') which separates the service channel (15) from the drainage channel (12), wherein the device permits delivery of fluid from the service channel (15) into the drainage channel (12) and prevents delivery of particles and liquid from the drainage channel (12) into the service channel (15) and wherein this device (4, 4') is arranged in the coupling part (1) at a location that allows a flow through the drainage channel (12) from the drainage connection (11) to the drainage orifice (16) without passage through the device (4, 4'),
**characterized in that** the pump-side drainage orifice (16) and the pump-side first service orifice (17) extend parallel to each other.

2. The coupling part as claimed in claim 1, wherein a connection channel (14) is present for connecting the second service orifice (17') to the drainage channel (12), and wherein the device (4, 4') is arranged in the area of this connection channel (14).

3. The coupling part as claimed in either of claims 1 and 2, wherein the service channel (15) and the drainage channel (12) extend approximately or exactly parallel to each other.

4. The coupling part as claimed in one of claims 1 through 3, wherein the device (4, 4') has a filter which is permeable to air and impermeable to liquid.

5. The coupling part as claimed in one of claims 1 through 4, wherein the device (4') has a one-way valve (45), in particular a duckbill valve.

6. The coupling part as claimed in one of claims 1 through 5, wherein the device (4') is a membrane, wherein the membrane acts as a filter and/or as a nonreturn valve.

7. The coupling part as claimed in claim 6, wherein the membrane (4') is round and has, in its peripheral area, at least one circumferential and closed sealing lip (46).

8. The coupling part as claimed in either of claims 6 and 7, wherein it has a main body (10) with a membrane seat (19) for receiving the membrane (4'), and a lid (13) with which the main body (10) in the area of the membrane seat (19) is closed from the outside in a leaktight manner.

9. The coupling part as claimed in claim 8, wherein the service channel (15) is arranged in the lid (13).

10. The coupling part as claimed in one of claims 6 through 9, wherein the membrane (4') forms a membrane surface that is approximately parallel to a longitudinal axis (L) of the drainage channel (12).

11. The coupling part as claimed in one of claims 6 through 10, wherein the membrane (4') is arranged asymmetrically with respect to a longitudinal center axis (L) of the drainage channel (12).

12. The coupling part as claimed in one of claims 1 through 11, wherein an injection and/or withdrawal opening (18) is also present, which connects an outer face of the coupling part (1) to the drainage channel (12).

13. The coupling part as claimed in one of claims 1 through 12, wherein two drainage channels (12, 12') and a single service channel (15) are present, wherein the service channel (15) is connected to both drainage channels (12, 12'), and wherein the device (4, 4') has a single membrane resp. a single filter that separates both drainage channels (12, 12') from the service line (15).

14. The coupling part as claimed in one of claims 1 through 4, wherein the device (4) has a tubular shape and extends coaxially with respect to the drainage channel (12).

15. The coupling part as claimed in claim 14, wherein the device (4) has a support body (40) and a filter element (44) that performs a filter function, wherein the filter element (44) is arranged on and/or in the support body (40).

## Revendications

1. Pièce d'accouplement (1) pour unité de tuyau de drainage destiné à aspirer des fluides corporels au moyen d'une pompe aspirante, la pièce d'accouplement comportant :
un raccord de drainage (11) situé côté patient, une embouchure de drainage (16) située côté pompe et destinée à être reliée à un tuyau (2) de drainage et un canal de drainage (12) qui relie mutuellement le raccord de drainage (11) et l'embouchure de drainage (16),
une première embouchure de service (17) située côté pompe, destinée à être reliée à un tuyau de service (3), une deuxième embouchure de service (17') qui se termine à l'intérieur de la pièce d'accouplement (1) et un canal de service (15) qui relie cette première et cette deuxième embouchure de service (17, 17') et
un dispositif (4, 4') qui sépare le canal de service (15) du canal de drainage (12),
le dispositif permettant d'apporter du fluide depuis le canal de service (15) jusque dans le canal de drainage (12) et empêchant un apport de particules et de liquide du canal de drainage (12) au canal de service (15),
ce dispositif (4, 4') étant disposé en un emplacement de la pièce d'accouplement (1) qui permet la traversée du canal de drainage (12) depuis le raccord de drainage (11) jusqu'à l'embouchure de drainage (16) sans traverser le dispositif (4, 4'), **caractérisée en ce que**
l'embouchure de drainage (16) située côté pompe et la première embouchure de service (17) située côté pompe s'étendent parallèlement l'une à l'autre.

2. Pièce d'accouplement selon la revendication 1, dans laquelle un canal de liaison (14) est prévu pour relier la deuxième embouchure de service (17') au canal de drainage (12), le dispositif (4, 4') étant disposé dans la région de ce canal de liaison (14).

3. Pièce d'accouplement selon l'une des revendications 1 ou 2, dans laquelle le canal de service (15) et le canal de drainage (12) s'étendent sensiblement ou exactement parallèlement l'un à l'autre.

4. Pièce d'accouplement selon l'une quelconque des revendications 1 à 3, dans laquelle le dispositif (4, 4') présente un filtre perméable à l'air et imperméable aux liquides.

5. Pièce d'accouplement selon l'une quelconque des revendications 1 à 4, dans laquelle le dispositif (4') présente une soupape (45) à une voie et en particulier une soupape en bec de canard.

6. Pièce d'accouplement selon l'une quelconque des revendications 1 à 5, dans laquelle le dispositif (4') est une membrane, la membrane agissant comme filtre et/ou comme soupape anti-retour.

7. Pièce d'accouplement selon la revendication 6, dans laquelle la membrane (4') est ronde et présente dans sa partie périphérique au moins une lèvre périphérique d'étanchéité (46) intrinsèquement fermée.

8. Pièce d'accouplement selon l'une quelconque des revendications 6 ou 7, qui présente un corps de base (10) doté d'un siège (19) de membrane qui reprend la membrane (4') et un couvercle (13) qui ferme de manière étanche vis-à-vis de l'extérieur le corps de base (10) dans la région du siège (19) de la membrane.

9. Pièce d'accouplement selon la revendication 8, dans laquelle le canal de service (15) est disposé dans le couvercle (13).

10. Pièce d'accouplement selon l'une quelconque des revendications 6 à 9, dans laquelle la membrane (4') forme une surface de membrane qui s'étend sensiblement en parallèle à un axe longitudinal (L) du canal de drainage (12).

11. Pièce d'accouplement selon l'une quelconque des revendications 6 à 10, dans laquelle la membrane (4') est disposée asymétriquement par rapport à un axe longitudinal central (L) du canal de drainage (12).

12. Pièce d'accouplement selon l'une quelconque des revendications 1 à 11, qui présente en outre une ouverture (18) d'injection et/ou de prélèvement qui relie le côté extérieur de la pièce d'accouplement (1) au canal de drainage (12).

13. Pièce d'accouplement selon l'une quelconque des revendications 1 à 12, dans laquelle deux canaux de drainage (12, 12') et un seul canal de service (15) sont prévus, le canal de service (15) étant relié aux deux canaux de drainage (12, 12') et le dispositif (4, 4') présentant une seule membrane ou un seul filtre qui séparent les deux canaux de drainage (12, 12') du conduit de service (15).

14. Pièce d'accouplement selon l'une quelconque des revendications 1 à 4, dans laquelle le dispositif (4) a une configuration tubulaire et s'étend coaxialement par rapport au canal de drainage (12).

15. Pièce d'accouplement selon la revendication 14, dans laquelle le dispositif (4) présente un corps de soutien (40) et un élément de filtre (44) qui exerce une fonction de filtration, l'élément de filtre (44) étant disposé sur et/ou dans le corps de soutien (40).
